# DEMANDE DE BREVET EUROPEEN

(11) **EP 1 443 108 A2**
(43) Date de publication de la demande: **04.08.2004**
(21) Numéro de dépôt: 04290063.9
(22) Date de dépôt: 09.01.2004
(51) Int. Cl.: C12N 15/06

(54) **Procédés de préparation d'un épiderme reconstruit supplémenté en céramide 7 ou 5.5, composition à base de vésicules lamellaires lipidiques incorporant un dérivé du céramide 7 et/ou 5.5 et son utilisation**

(30) Priorité: 30.01.2003 FR 0301058; 30.01.2003 FR 0301059
(71) Demandeur: L'OREAL, 75008 Paris (FR)
(72) Inventeur: Chopart, Mélanie, 75012 Paris (FR); Castiel, Isabelle, 06000 Nice (FR); Simonnet, Jean-Thierry, 94240 Cachan (FR)
(74) Mandataire: Barbier, Denis Robert

(57) **Abrégé**

La présente invention se rapporte à un procédé de préparation d'un épiderme reconstruit ou d'un équivalent de peau supplémenté en au moins un dérivé du céramide 7 et/ou 5.5.

L'invention se rapporte encore à un épiderme reconstruit ou un équivalent de peau contenant au moins un dérivé de la famille du céramide 5.5.

L'invention se rapporte encore à une composition comprenant une dispersion, dans une phase aqueuse externe, de vésicules formées par des phases lamellaires lipidiques séparées les unes des autres par des couches hydrophiles, lesdites phases lamellaires comprenant au moins un lipide amphiphile, et au moins un dérivé de la famille du céramide 7 et/ou du céramide 5.5 inclus dans lesdites phases lamellaires lipidiques, ainsi qu'à son utilisation pour renforcer la fonction barrière des épidermes humains normaux, et améliorer la fonction barrière des peaux sèches et des peaux reconstruites ou équivalents de peau.

## Description

La présente invention se rapporte à un procédé de préparation d'un épiderme reconstruit ou d'un équivalent de peau supplémenté en au moins un dérivé du céramide 7 et/ou 5.5, comprenant l'introduction d'au moins un dérivé du céramide 7 et/ou 5.5 dans le milieu de culture dudit épiderme reconstruit ou dudit équivalent de peau et/ou l'application topique sur ledit épiderme reconstruit ou ledit équivalent de peau d'une composition à base de vésicules lamellaires lipidiques incorporant au moins un dérivé de la famille du céramide 7 et/ou 5.5.

L'invention se rapporte encore à un épiderme reconstruit ou un équivalent de peau contenant au moins un dérivé de la famille du céramide 5.5.

La présente invention se rapporte également à une composition comprenant une dispersion, dans une phase aqueuse externe, de vésicules formées par des phases lamellaires lipidiques séparées les unes des autres par des couches hydrophiles, lesdites phases lamellaires comprenant au moins un lipide amphiphile, et au moins un dérivé de la famille du céramide 7 et/ou du céramide 5.5 inclus dans lesdites phases lamellaires lipidiques, ainsi qu'à l'utilisation de la dite composition pour renforcer la fonction barrière des épidermes humains normaux, et améliorer la fonction barrière d'un épiderme présentant un déficit même léger en céramides à base 6-hydroxy-4-sphingénine, notamment des peaux sèches et des peaux reconstruites ou équivalents de peau.

La peau humaine est constituée de deux compartiments à savoir un compartiment profond, le derme, et un compartiment superficiel, l'épiderme.
L'épiderme est en contact avec l'environnement extérieur. Son rôle consiste à protéger l'organisme de la déshydratation et des agressions extérieures, qu'elles soient chimiques, mécaniques, physiques ou infectieuses.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Les cellules constituant l'épiderme sont délimitées par un domaine lipidique. Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides.

Ces lipides sont organisés en phase cristal liquide lamellaire spécifiques dont l'intégrité dépend non seulement de la qualité des fractions présentes mais aussi de leur proportion respective. Cette structure lamellaire des lipides du domaine lipidique de l'épiderme est responsable de la fonction barrière épidermique.

Les lipides épidermiques sont synthétisés principalement dans l'épiderme vivant. Ils sont principalement constitués de phospholipides, de céramides (ou sphingolipides), de cholestérol, d'acides gras libres, de triglycérides, d'esters du cholestérol et d'alcanes.

Les céramides sont un des constituants essentiels des lipides épidermiques, permettant d'assurer en partie, la structure cristal liquide lamellaire de ceux ci, mais aussi la fonction barrière de l'épiderme.

Les céramides sont composés d'une base sphingoïde qui peut être de quatre types, sphinganine, sphingénine, phytosphingosine et 6-hydroxy-4-sphingénine, et d'un acide gras qui peut être saturé, α hydroxylé ou ? estérifié. Les différentes combinaisons possibles entre bases et acides gras conduisent à une dizaine de céramides répertoriés par Robson,K.J.; Stewart,M.E.; Michelsen,S.; Lazo,N.D.; Downing,D.T., 6-hydroxy-4-sphingenine in human epidermal ceramides, dans J. Lipid Res. 1994 35 :2060-2068; et Chopart M., Castiel-Higounenc I., Arbey E., Guey C., Gaetani Q., Schmidt R., The Normal Human stratum corneum: a new ceramide profile, Perspectives in Percutaneous Penetration, 8th International Conference, Antibes Juan-Les Pins - France, April 2-6, 2002.

Des modèles plus ou moins proches de la peau humaine ont pu être mis au point. On peut citer par exemple les modèles décrits dans les demandes de brevets EP-A-285471, EP-A-285474, EP-A-789074, EP-A-502172, EP-A-418035, WO-A-9116010, EP-A-197090, EP-A-20753, FR-A-2665175, FR-A-2689904 et FR-A-2792650.

De manière très générale, les modèles de peau reconstruite décrits dans ces documents comprennent des kératinocytes humains associés ou non à d'autres cellules de la peau comme les mélanocytes et/ou les cellules de Langerhans, déposés sur un support, souvent un équivalent de derme, et cultivés dans des conditions telles qu'ils entrent dans un programme de différenciation aboutissant à la formation d'un équivalent d'épiderme. Les équivalents de derme décrits à ce jour sont soit des membranes artificielles comme par exemple les filtres de marque Millipore, des substituts sous-cutanés à base de collagène, du plastique ou tout autre support compatible avec la viabilité cellulaire, soit des supports plus élaborés pour les rendre plus proches du derme naturel, comme le derme préalablement désépidermisé ou des lattices mixtes collagène/fibroblastes. Dans les lattices mixtes collagène/fibroblastes l'association de collagène natif et de fibroblastes humains isolés conduit à l'obtention d'un équivalent de derme mimant un derme qui n'a pas subi l'action du temps.

Les modèles de peaux reconstruites présentent généralement une fonction barrière déficiente (M. Ponec, P.J.J. Wauben-Penris, A. Burger, J. Kempenarr, H. E. Boddé, Skin Pharmacol 1990; 3: pp.126-135). Cette déficience est en grande partie due à d'importantes modifications du profil céramidique de ce modèle qui ont été observées par rapport à un épiderme humain normal.

En outre, Il est connu de l'art antérieur que les peaux atopiques, xérotiques, et âgées peuvent être liées à une diminution de la synthèse des céramides 1 et/ou 3.
On peut citer par exemple les documents suivants:
- Imokawa *et al.* dans *J Invest Dermatol,* 96 (4) : 523-6, 1991 décrivent que les céramides interviennent dans la fonction barrière et que leur synthèse, en particulier celle du céramide 1, est diminuée dans le cas des peaux atopiques et xérotiques.
- Di Nardo *et al.* dans *Acta Derm Venereol,* 78 (1) : 27-30, 1998) décrivent la dermite atopique comme une peau facilement irritable et sèche, dans laquelle la fonction barrière est altérée. Les auteurs ont montré qu'une diminution de la synthèse des céramides 1 et 3 peut être la cause de la peau sèche et de l'altération de la fonction barrière dans la dermatite atopique;
- Rogers *et al.* dans *Arch. Dermatol. Res.* 288 :765-770, 1996 décrivent une diminution de la synthèse des céramides, en particulier du céramide 1, ce qui contribue, dans les peaux âgées, à une perturbation de la fonction barrière et à la xérose, particulièrement durant les mois d'hiver.
- Rogers *et al.* dans *J. Invest. Dermatol.* 100 :510, 1993 décrivent que la structure multicouche lipidique des peaux sèches est perturbée, et que cette perturbation est accompagnée d'une augmentation des acides gras libres et d'une diminution. des céramides.

Afin d'améliorer la peau âgée, la peau sèche et la sensibilité cutanée on connaît déjà des compositions, contenant un précurseur de la synthèse des céramides choisi parmi : les bases sphinganine, shingosine, les amides des acides gras et de la vitamine B₃ comme stimulateur de la synthèse des céramides (WO99/47114 et des compositions contenant contenant un intermédiaire des voies de synthèse ou précurseur des céramides choisi parmi les acides gras, les base sphinganine et sphingosine. et de la vitamine A comme stimulateur de la synthèse des céramides (WO94/23694).

M. Ponec *et al.* dans Skin Pharmacol 1990; 3: pp.126-135 décrivent par ailleurs que les modèles de peaux reconstruites présentent généralement une fonction barrière déficiente. Cette déficience est en grande partie due à d'importantes modifications du profil céramidique de ce modèle qui ont été observées par rapport à un épiderme humain normal.

Il est également connu du document FR 2 811 556 d'utiliser de la 6-hydroxy-4-sphingénine pour augmenter la fonction barrière des peaux reconstruites et pour renforcer la barrière lipidique de l'épiderme, notamment de la peau sèche et/ou rugueuse et/ou abîmée, et/ou rétablir ou maintenir l'intégrité du stratum corneum, et/ou améliorer l'aspect de surface et/ou l'hydratation de la peau, et/ou protéger la peau, notamment les peaux sèches et rugueuses, et/ou comme agent de nutrition essentiel pour les matières kératiniques (peau, cheveu, cil, ongle), et/ou renforcer la barrière lipidique de la peau reconstruite, ou équivalent de peau et/ou améliorer et/ou maintenir le contenu lipidique de l'épiderme humain, *in vivo* et *in vitro,* et/ou améliorer la qualité et les propriétés telles que le contenu en lipides et/ou propriété barrière, des épidermes reconstruits et/ou des cultures de cellules épidermiques..

Toutefois, en utilisant de la 6-hydroxy-4-sphingénine on ne peut contrôler la nature du céramide qui va être obtenu *in situ* suite à son association avec un acide gras au niveau de l'épiderme. La 6-hydroxy-4-sphingénine est en effet la base sphingoïde constitutive de plusieurs céramides: céramides STAR, 4, 5.5 et 7 ("The Normal Human stratum corneum: a new ceramide profile" M. Chopart *et al.* Prospectives in Percutaneous Penetration, 8th Internationak Conference Antibes Juan-Les Pins, France April 2-6, 2002 et le document JP2000/143,598 de Kanebo). En outre, une peau reconstruite est une structure fragile qui ne peut être maintenue en survie que durant un temps limité (environ 1 mois). Tout gain de temps permettant à une peau reconstruite d'acquérir une fonction barrière est donc un paramètre important en rapport à sa durée de vie.

Afin d'améliorer le profil lipidique des épidermes reconstruits, il est également connu d'ajouter de l'acide ascorbique ou vitamine C (*J. Invest. Dermatol.* 109 :348-355, 1997) ou des dérivés d'acide ascorbique (FR 2 807 320) dans le milieu de culture.
Toutefois, en raison de sa structure chimique (alpha-cétolactone), l'acide ascorbique est très sensible à certains paramètres de l'environnement comme la lumière, la chaleur et les milieux aqueux, en particulier les milieux alcalins et/ou aérobies. En raison de ces problèmes de stabilité, il est nécessaire d'utiliser de fortes concentrations d'acide ascorbique pour observer l'effet sur la peau d'une composition le contenant. En outre, en apportant dans le milieu de culture de l'acide ascorbique ou l'un de ses dérivés on ne peut pas contrôler la nature exacte du céramide qui va être synthétisé *in situ.*

Il subsiste donc le besoin de disposer d'autres procédés permettant d'obtenir plus rapidement des modèles de peaux reconstruites dont le profil céramidiques est amélioré, avec une fonction barrière se rapprochant de l'épiderme humain normal.

La demanderesse a maintenant découvert un nouveau procédé de préparation d'un épiderme reconstruit ou d'un équivalent de peau supplémenté en au moins un dérivé du céramide 7 et/ou 5.5, comprenant l'introduction dans le milieu de culture au moins un dérivé du céramide 7 et/ou 5.5 et/ou l'application topique sur ledit épiderme reconstruit ou ledit équivalent de peau d'une composition à base de vésicules lamellaires lipidiques incorporant au moins un dérivé de la famille du céramide 7 et/ou 5.5.

Grâce au nouveau procédé de préparation on peut maintenant obtenir un équivalent de peau, ou une peau reconstruite, supplémenté en au moins un dérivé de céramide 5.5 et/ou 7 de formule (I), de préférence un dérivé de céramide 7 de formule (I), en contrôlant exactement la nature du céramide qui voit sa proportion augmentée dans ledit équivalent de peau ou ladite peau reconstruite.

La demanderesse a maintenant également trouvé qu'une composition à base de vésicules lamellaires lipidiques incorporant au moins un dérivé de la famille du céramide 7 et/ou du céramide 5.5 permet d'améliorer la fonction barrière d'un épiderme présentant un déficit en céramides à base 6-hydroxy-4-sphingénine. Ceci est particulièrement avantageux car il a été montré dans le cadre de l'invention que les peaux sèches et les peaux reconstruites (étude réalisée sur le modèle EPISKINT™) présentent un déficit en céramides les plus polaires qui se traduit par un déficit en bases sphingoïdes 6-hydroxy-4-sphingénine (appelée aussi 6-hydroxy-4-sphingosine).

Ainsi, Un premier objet de la présente invention se rapporte à un procédé de préparation d'un épiderme reconstruit ou d'un équivalent de peau supplémenté en au moins un dérivé du céramide 7 et/ou 5.5 de formule (I), comprenant l'introduction d'au moins un dérivé du céramide 7 et/ou 5.5 dans le milieu de culture dudit épiderme reconstruit ou dudit équivalent de peau et/ou l'application topique sur la surface dudit épiderme reconstruit ou dudit équivalent de peau d'une composition à base de vésicules lamellaires lipidiques incorporant au moins un dérivé de la famille du céramide 7 et/ou 5.5 de formule (I) suivante: 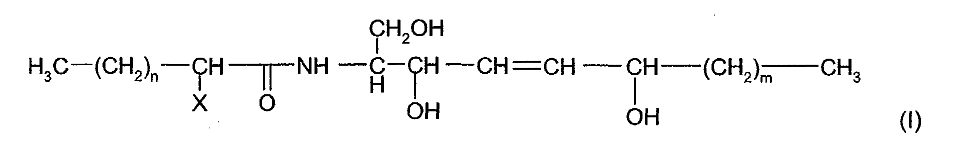 dans laquelle :
- X représente un atome d'hydrogène ou un groupement hydroxyle (OH); avantageusement un groupement hydroxyle ;
- n représente un nombre entier allant de 19 à 29, de préférence de 21 à 27, avantageusement égal à 21, 22 ou 23;
- m représente un nombre entier allant de 9 à 19, de préférence de 9 à 15, avantageusement égal à 11, 12 ou 13.

Les dérivés de céramide 5.5 de formule (I) sont ceux pour lesquels X représente un atome d'hydrogène.
Les dérivés de céramide 7 de formule (I) sont ceux pour lesquels X représente un groupement hydroxyle.

Suivant un mode de réalisation préféré les composés de formule (I) sont ceux pour lesquels n va de 21 à 27 et m va de 9 à 15.

Suivant un autre mode de réalisation préféré les composés de formule (I) sont ceux pour lesquels n est égal à 21, 22 ou 23 et m est égal à 11, 12 ou 13.
Le céramide 7, tel que décrit par Robson *et al.,* dans J. Lipid Res. 1994 35 :2060-2068, correspondant au composé de formule (I) pour lequel n égal à 21 et m égal à 11, est particulièrement préféré suivant l'invention.

Suivant les procédés de l'invention, la concentration d'au moins un dérivé de formule (I) introduit dans le milieu de culture va de 10 g/l à 10⁻⁶ g/l, de préférence de 1g/l à 10⁻⁵ g/l, avantageusement de 10⁻¹ g/l à 10⁻⁵ g/l de milieu de culture.

L'introduction dans le milieu de culture d'au moins un dérivé de céramide 5.5 et/ou 7 de formule (I) peut se faire par différents procédés qui seront choisis de manière telle que le dérivé de céramide 5.5 et/ou 7 de formule (I) soit au final solubilisé dans le milieu de culture. En effet, du fait de la structure lipophile des dérivés de céramide 5.5 et/ou 7 de formule (I), on comprend que cette dissolution dans le milieu de culture ne soit pas à priori évidente. Suivant une première variante, le dit dérivé est dissout au préalable dans un solvant. Dans une autre variante, le dit dérivé est associé au préalable avec une autre molécule capable de le véhiculer. Une autre variante consiste à réalisée au préalable une composition à base de vésicules lamellaires lipidiques incorporant au moins ledit dérivé de la famille du céramide 7 et/ou 5.5. Ces compositions, associations et/ou solvatations ainsi obtenues au préalable sont alors introduites dans le milieu de culture.

Le solvant choisi doit être capable de solubiliser le céramide 7 et/ou 5.5 de formule (I). La solution obtenue est alors introduite dans le milieu de culture. La quantité de solvant introduite dans le milieu de culture est telle qu'elle ne gêne pas le développement normal de l'épiderme et son homéostasie. Avantageusement, le céramide 7 et/ou 5.5 de formule (I) peut être dissout dans de l'éthanol ou le DMSO à la concentration voulue, le rapport finale en solvant introduit dans le milieu de culture ne devant pas dépasser 1/1000.

Le procédé suivant l'invention peut également consister à introduire dans le milieu de culture une association préparée au préalable contenant au moins un dit dérivé et au moins une molécule capable de véhiculer et de rendre biodisponible le dit dérivé au sein de l'épiderme reconstruit ou dudit équivalent de peau à partir du milieu de culture.

Les molécules capables de véhiculer et de rendre biodisponible le dit dérivé sont avantageusement choisies parmi le BSA (Bovine Sérum Albumine) ou albumine de sérum de boeuf et/ou un composé de la famille des cyclodextrines ou un dérivé de la famille des cyclodextrines permettant un transport et une solubilisation dans le milieu de culture de façon similaire.

Lorsque le céramide 7 et/ou 5.5 de formule (I) est associé à du BSA, le ratio molaire par rapport au BSA va par exemple de 1/1000 à 1/3, préférentiellement de 1/100 à 1/5. La concentration de BSA pouvant être introduite dans le milieu de culture doit être inférieure ou égale à 100 µmol/l, de préférence de 0.5 µmol/l à 100 µmol/l, préférentiellement de 10 µmol/l à 50 µmol/l.

Lorsque le céramide 7 et/ou 5.5 de formule (I) est associé à au moins un composé de la famille des cyclodextrines, avantageusement le HPBCD ou 12-hydroxypropyl-β-cyclodextrin, ou un dérivé de la famille des cyclodextrines permettant un transport et une solubilisation dans le milieu de culture de façon similaire, la concentration approximative de céramide 7 et/ou 5.5 de formule (I) pourra par exemple être de 0.01 nmol/µl à 100 nmol/µl, préférentiellement de 0.1 nmol/µl à 10 nmol/µl et plus particulièrement entre 1 nmol/µl et 5 nmol/µl d'une solution à 50% en cyclodextrines (dilué dans de l'eau). Cette concentration sera bien sûr adaptée en fonction du dérivé choisi et de la composition annexe du milieu de culture lui-même. Cette préparation est ensuite introduite dans le milieu de culture, le volume ainsi introduit étant adapté à la concentration finale voulue en céramide 7 et/ou 5.5 de formule (I).

Les associations précédemment décrites formés par le céramide 7 et/ou 5.5 de formule (I) et un véhicule, peut comprendre en outre :
- un agent antioxydant, avantageusement le D.L α Tocophérol acétate à une concentration inférieure ou égale à 50 µmol/l, de préférence de 0.5µmol/l à 50 µmol/l de milieu de culture, avantageusement 21 µmol/l de milieu de culture, et/ou
- un agent transporteur cellulaire, avantageusement la L-Carnitine à une concentration inférieure ou égale à 100 µmol/l, de préférence de 0.5 à 100 µmol/l, avantageusement 10 µmol/l de milieu de culture.

Lorsque le procédé suivant l'invention consiste à introduire dans le milieu de culture une composition à base de vésicules lamellaires lipidiques incorporant au moins un dérivé de la famille du céramide 7 et/ou 5.5, ladite composition comprend de préférence une dispersion, dans une phase aqueuse externe, de vésicules formées par des phases lamellaires lipidiques séparées les unes des autres par des couches hydrophiles, lesdites phases lamellaires comprenant au moins un lipide amphiphile, et au moins ledit dérivé de formule (I) inclus dans lesdites phases lamellaires lipidiques.

Les concentrations précitées pourront être adaptées (augmentées comme diminuées) de manière à rester dans une gamme de concentration telle qu'elles ne gênent pas le développement normal de l'épiderme et son homéostasie.

Le milieu de culture selon l'invention est un milieu bien connu de l'homme du métier. Il s'agit en particulier d'un milieu tel décrit dans l'un des documents suivants EP-A-285471, EP-A-285474, EP-A-789074, EP-A-502172, EP-A-418035, WO-A-9116010, EP-A-197090, EP-A-20753, FR-A-2665175, FR-A-2689904 et FR-A-2792650 FR 2 811 556.

Suivant un autre mode de réalisation du procédé suivant l'invention, la peau reconstruite supplémentée en au moins un céramide 7 et/ou 5.5 de formule (I) est obtenue par application topique sur la surface des épidermes en culture d'une composition à base de vésicules lamellaires lipidiques incorporant au moins un dérivé de la famille du céramide 7 et/ou 5.5.
Avantageusement, la dite composition comprend une dispersion, dans une phase aqueuse externe, de vésicules formées par des phases lamellaires lipidiques séparées les unes des autres par des couches hydrophiles, lesdites phases lamellaires comprenant au moins un lipide amphiphile, et au moins ledit dérivé de formule (I) inclus dans lesdites phases lamellaires lipidiques.

Un autre objet de la présente invention se rapporte à une composition comprenant une dispersion, dans une phase aqueuse externe, de vésicules formées par des phases lamellaires lipidiques séparées les unes des autres par des couches hydrophiles, lesdites phases lamellaires comprenant au moins un lipide amphiphile, et au moins un dérivé de formule (I) telle que définie précédemment, inclus dans lesdites phases lamellaires lipidiques.

La composition suivant l'invention peut être utilisée de manière toute particulière pour le soin de la peau. Notamment, les compositions selon l'invention peuvent permette d'améliorer la fonction barrière des peaux sèches, des peaux rugueuses et/ou abîmées et/ou âgées et/ou sensibles et/ou atopiques, des peaux reconstruites ou équivalents de peaux, ainsi que tout épiderme présentant un déficit même léger en céramides à base 6-hydroxy-4-sphingénine ou de renforcer encore d'avantage la fonction barrière des peaux humaines normales.

Un autre objet de l'invention se rapporte donc à l'utilisation de la dite composition pour :
- renforcer la fonction barrière des épidermes humains normaux, et/ou
- renforcer la fonction barrière des peaux reconstruites ou équivalents de peaux, avantageusement la peau reconstruite est le modèle EPISKIN™ , et/ou
- améliorer la fonction barrière d'un épiderme présentant un déficit, même léger, en céramides à base 6-hydroxy-4-sphingénine, de préférence des peaux sèches, des peaux rugueuses et/ou abîmées et/ou âgées et/ou sensibles, et/ou
- rétablir ou maintenir l'intégrité du stratum corneum, et/ou
- améliorer l'aspect de surface et/ou l'hydratation de la peau, et/ou
- améliorer et/ou maintenir le contenu lipidique de l'épiderme humain, *in vivo* et *in vitro,* et/ou
- améliorer la qualité et les propriétés telles que le contenu en lipides et/ou propriété barrière, des épidermes reconstruits et/ou des cultures de cellules épidermiques.

Un autre objet de l'invention se rapporte encore à l'utilisation de la composition selon l'invention pour la fabrication d'une composition destinée au traitement des peaux atopiques.

Il est bien établi que les peaux reconstruites représentent un modèle proche de la peau humaine. Dès lors, une composition suivant l'invention, capable d'augmenter la fonction barrière des peaux reconstruites, trouve également un intérêt pour les épidermes humains normaux, ainsi que pour tout épiderme présentant un déficit même léger en céramides à base 6-hydroxy-4-sphingénine notamment des épidermes des peaux sèches et/ou rugueuses et/ou abîmées et/ou âgées et/ou sensibles, et/ou des peaux atopiques, en maintenant ou en rétablissant un profil céramidique tel qu'il permet d'augmenter ou de maintenir leur fonction barrière.

Un autre objet de l'invention se rapporte à un procédé de traitement cosmétique d'embellissement ou d'hydratation de la peau humaine, caractérisé à ce qu'il consiste à appliquer topiquement sur la peau une composition telle que précédemment définie.

Par vésicule ou dispersion vésiculaire on entend une dispersion de lipides amphiphiles formant au contact de l'eau ou d'un milieu hydrophile, des particules dont le coeur est hydrophile (eau ou mélange hydrophile) et dont la paroi est constituée de bicouches de type cristal liquide lamellaire. Ces vésicules sont communément appelées Liposomes. Ils sont principalement constitués de phospholipides, naturels ou synthétiques, hydrogénés ou non. Les Niosomes, quant à eux, sont constitués de tensioactifs non ioniques, éventuellement associés à du cholestérol et/ou un tensioactif ionique.

Les vésicules selon l'invention sont formées par, ou comprennent, de un à vingt cinq feuillets de phases lamellaires sensiblement concentriques de type bimoléculaires.

Les vésicules suivant l'invention peuvent être soit des niosomes du type de ceux décrits dans les demandes EP 0 958 856, EP 0 582 503, EP 0 455 528, EP 0 043 327, soit des liposomes de type classique. Les dérivés de formule (I) suivant l'invention deviennent, dans ce type de structure, l'un des constituants des phases lamellaires.

Le taux de dérivé de formule (I) va de 0.001% à 30%, préférentiellement 0.001% à 10%, avantageusement de 0.001% à 5% par rapport au poids total de la composition lipidique constituant les vésicules.

Le rapport en poids entre la quantité de phase lipidique et la quantité de phase aqueuse de la dispersion va de 1/1000 à 300/1000.

Les dispersions vésiculaires selon l'invention peuvent être préparées suivants de nombreux procédés bien connus de l'homme du métier.
Par exemple, selon un premier procédé, on dissout tous les lipides amphiphiles, y compris les dérivés de formule (I) suivant l'invention, dans un solvant volatil, on forme un film mince lipidique sur les parois d'un flacon par évaporation du solvant, puis on reprend le film lipidique dans une solution aqueuse d'octylglucoside pour former des micelles mixtes octylglucoside/lipides vésiculaires. Cette solution est ensuite dialysée contre de l'eau distillée. Les liposomes se forment au fur et à mesure que l'octylglucoside est dialysé. Ce procédé est particulièrement adapté lorsque l'on ne dispose que d'une très faible quantité de dérivés de formule (I).
Cette méthode n'est cependant pas limitative et les autres méthodes utilisées pour former des dispersions vésiculaires (liposomes) sont envisageables (Bangham, par injection d'éthanol, par fusion, par « reverse phase »...). On peut encore citer la méthode décrite dans le brevet EP 0 582 503 B1.

Tel que mis en évidence sur les peaux reconstruites, la composition selon l'invention à base de vésicules lamellaires lipidiques incorporant au moins un dérivé de formule (I) permet d'améliorer la qualité de l'épiderme là où la simple application topique dudit dérivé de formule (I) n'apporte aucune amélioration desdits épidermes.

L'incorporation d'au moins un dit dérivé de formule (I) dans les phases lamellaires lipidiques permet d'obtenir l'effet recherché sur l'amélioration de la fonction barrière de l'épiderme reconstruit tout en prenant en compte les caractéristiques intrinsèques des dérivés de formule (I) : grosses molécules, difficiles à formuler et à stabiliser.

Une dispersion vésiculaire selon l'invention permet en outre d'améliorer la biodisponibilité des dérivés de formule (I) au sein des couches de l'épiderme. Ladite biodisponibilité est améliorée car la formule de la dispersion vésiculaire suivant l'invention représente une formule proche des structures lipidiques multicouches de l'épiderme qui représentent la cible desdits dérivés de formule (I).
En outre, les dérivés de formule (I) présentent un caractère amphiphile marqué ce qui présente l'avantage de faciliter leur incorporation au sein de l'épiderme.

Ainsi, la dispersion vésiculaire suivant l'invention contenant au moins un dérivé de formule (I) permet de contrôler la nature du céramide qui est apporté spécifiquement au sein des structures lipidiques multicouches de l'épiderme.

Par biodisponibilité, on entend au sens de l'invention, la pénétration d'un actif dans la peau pour que celui-ci soit biologiquement disponible pour les éléments vivants de la peau, et en particulier l'épiderme. Ainsi, l'augmentation de la biodisponibilité d'un actif a pour effet d'augmenter le taux d'actif qui va arriver jusqu'à l'épiderme vivant.

En outre, les compositions selon l'invention permettent de rendre disponible le céramide 5.5 et/ 7 directement au sein de la couche cornée là où sans formulation adéquate, le dit céramide ne pénètre pas la couche cornée. Ainsi, les compositions selon l'invention permettent de combler directement dans la couche cornée un déficit en céramide 5.5 et/ou 7.

La composition peut être appliquée topiquement sur la surface des épidermes en culture en une quantité de 0.5 µl à 10 µl, préférentiellement 1 µl à 5 µl et plus particulièrement de 2 µl par cm² de surface d'épiderme reconstruit. La composition est ensuite généralement rendue homogène à la surface des épidermes par étalement à l'aide d'une spatule.

Les dérivés de formule (I) suivant l'invention peuvent être isolés par CCM à partir de prélèvements lipidiques réalisés par des méthodes non invasives sur des volontaires sains suivant une méthode bien connue de l'homme du métier. Ces prélèvements lipidiques sont ensuite soumis à un traitement préparatif et analytique permettant de d'isoler les dérivés de formule (I) des autres lipides et de les purifier. Ces méthodes sont par exemple décrites dans le document JP2000/143,598 ou M. Chopart *et al.* Prospectives in Percutaneous Penetration, 8th International Conference Antibes Juan-Les Pins, France April 2-6, 2002 et le document JP2000/143,598 de Kanebo).

Les dérivés de formule (I) peuvent être également préparés selon des procédés de synthèse organique classique, par exemple par une réaction de condensation entre un acide gras de formule (II) et une base de formule (III), de préférence la base 6-hydroxy-4-sphingénine. 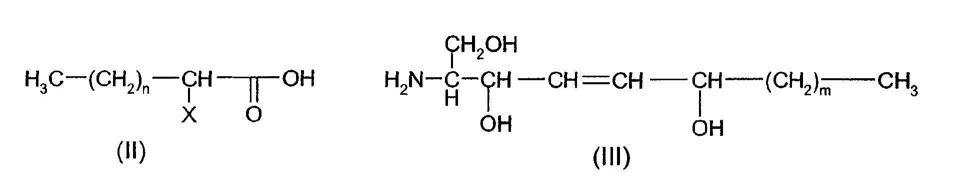 X, n et m conservent les définitions données précédemment.

La base 6 hydroxy-4-sphingénine peut être préparée selon des procédés de synthèse décrits et bien connus de l'homme du métier, par exemple tels que décrits ou inspirés de "Mendeleev, Commun. 108-110, 1992" ou "Tetrahedron Letters, 34, n°7, 1191-1194, 1993".
Les acides gras sont largement disponibles commercialement.
Une autre méthode de synthèse organique classique, décrite dans le document JP2000/143,598, consiste en une réaction d'oxydation en position 6 de la base à partir d'un précurseur non oxydé.

Le dérivé de formule (I) pourra être introduit à tout moment de la vie de l'équivalent de peau (au maximum 2 mois) qu'il soit à l'état d'immersion ou d'émersion suivant l'un au moins des procédés suivant l'invention par application topique et/ou par introduction dans le milieu de culture. Préférentiellement, le céramide 7 et/ou 5.5 de formule (I) pourra être apporté à l'équivalent de peau entre le 1° jour de culture et le trentième jour de culture et plus particulièrement entre le quatrième jour et le vingt-et-uniène jour.

La supplémentation directe dans le milieu de culture pourra avoir lieu à chaque changement du milieu de culture qui intervient généralement tous les deux jours. Cette supplémentation pourra être augmentée ou diminuée du fait des changements du milieu de culture intervenant en fonction de l'effet voulu et des impératifs des études ou de certains procédés de fabrication.
La supplémentation par voie topique pourra avoir lieu préférentiellement tous les deux jours. Là encore cette fréquence sera adaptée, allant d'une supplémentation toutes les heures à toutes les semaines, en fonction des effets voulus et des impératifs des études ou de certains procédés de fabrication.

Suivant un mode de réalisation préféré de l'invention, le procédé est mis en oeuvre sur le modèle d'épiderme reconstruit EPISKIN^{TM}.

Les composés de formule (I) tels que définis précédemment présentent donc le grand avantage de mettre à la disposition des chercheurs, un équivalent de peau nouveau, supplémenté en au moins un dérivé de formule (I). Suivant le procédé de l'invention, la quantité de céramide 7 et/ou 5.5 pouvant être introduite au sein du stratum corneum de la peau reconstruite représente de 0.1 µg à 50 µg par mg de stratum corneum, avantageusement de 0.5µg à 15 µg par mg de stratum corneum.

De préférence, l'équivalent de peau ou peau reconstruite supplémentée en au moins un dérivé de la famille du céramide 5.5 ou 7 de formule (I) obtenu suivant l'un des procédés de l'invention contient une quantité de céramide 5.5 ou 7 de formule (I) supérieure à 1% des céramides totaux.

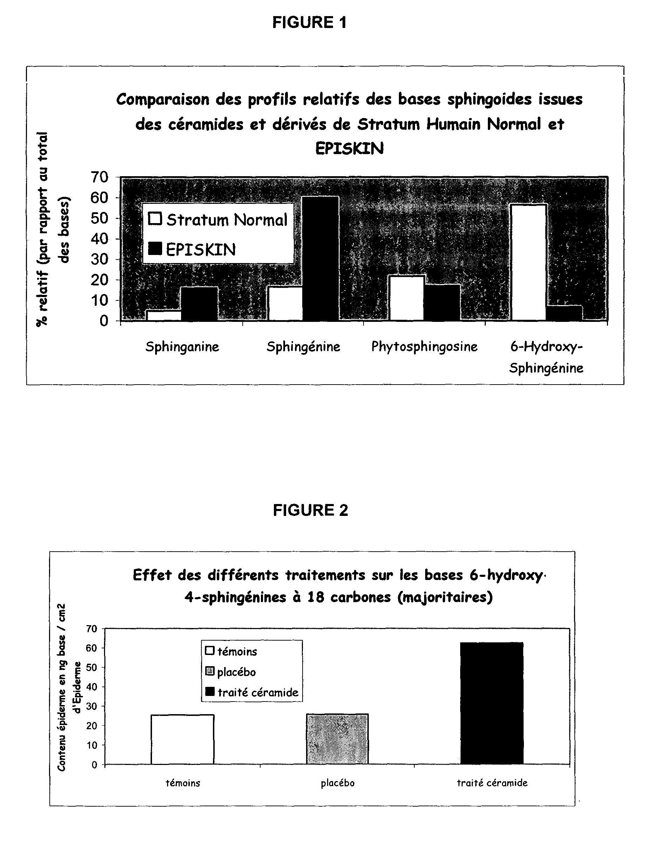

La présente invention est également basée sur l'observation de la demanderesse suivant laquelle les peaux reconstruites présentent un net déficit en céramides les plus polaires qui se traduit par un déficit en bases sphingoïdes 6-hydroxy-4-sphingénine composant ces céramides au profit de la base 4-sphingénine (appelée aussi sphingosine) essentiellement. Les résultats obtenus sont présentés sur la Figure 1. La Figure 1 présente une comparaison des profils relatifs des bases sphingoides issues des céramides et dérivés de Stratum Humain Normal et EPISKIN^{TM}.

La demanderesse a découvert que les dérivés de la famille du céramide 5.5 de formule (I) avec X= H tel que défini précédemment permettent de renforcer la fonction barrière de la peau reconstruite, aussi appelée équivalent de peau.

En effet, on a constaté que la supplémentation desdites peaux reconstruites en au moins un dérivé de formule (I) avec X= H tel que défini précédemment permet d'améliorer l'aspect et les propriétés barrière de ces dites peaux, et de les rendre plus proches structurellement des peaux normales.

On peut ainsi obtenir un nouveau équivalent de peau, ou nouvelle peau reconstruite, contenant au moins un dérivé de céramide 5.5 de formule (I) avec X= H tel que défini précédemment.

Un autre objet de la présente invention se rapporte donc à un équivalent de peau ou peau reconstruite contenant au moins un dérivé de formule (I) avec X = H tel que défini précédemment, n et m conservant par ailleurs les mêmes définitions que celles données précédemment. De préférence, l'équivalent de peau ou peau reconstruite contient une quantité de céramide 5.5 de formule (I) supérieure à 1 % des céramides totaux.

Le dit équivalent de peau ou la dite peau reconstruite contenant au moins un dérivé de formule (I) avec X = H peut être obtenu par la mise en oeuvre de l'un des procédés tels que décrits précédemment.

Les lipides amphiphiles constitutifs des dispersions vésiculaires suivant l'invention sont bien connus de l'homme du métier. Par exemple, ces lipides amphiphiles peuvent être à base de phospholipides naturels ou de synthèse, hydrogénés ou non et/ou de tensioactifs non ioniques pouvant être associés ou non à du cholestérol.
Les lipides amphiphiles ioniques, cationiques ou anioniques, ou non ioniques préférés suivant l'invention sont choisis parmi ceux décrits dans les documents EP 0 582 503 A1, FR 2 485 921, et FR 2 315 991.

Les lipides amphiphiles anioniques (B) préférés sont choisis dans le groupe formé par:
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides ;
- les dérivés alkylsulfoniques notamment de formule (X) : 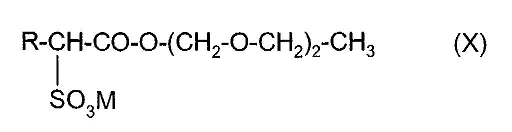 dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium ; et leurs mélanges.

Les lipides amphiphiles cationiques selon l'invention sont de préférence choisis dans le groupe formé par les sels d'ammonium quaternaire, les amines grasses et leurs sels.

Les sels d'ammonium quaternaires sont par exemple :
- ceux qui présentent la formule générale (XI) suivante; 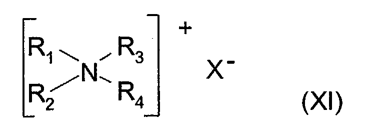 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (XII) suivante: 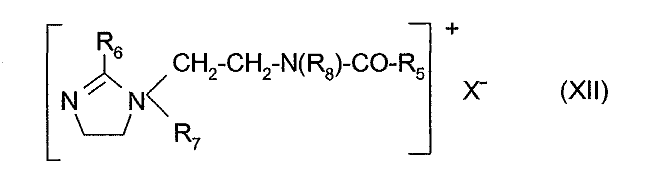 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- les sels de diammonium quaternaire de formule (XIII) suivante; 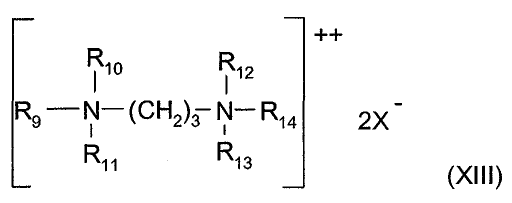 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester.

Les sels d'ammonium quaternaire contenant au moins une fonction ester utilisables selon l'invention sont par exemple ceux qui répondent à la formule (XIV) suivante : 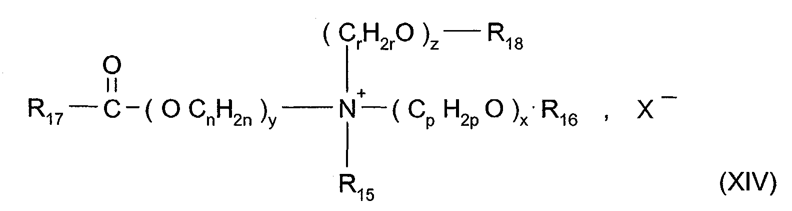 dans laquelle :
- R₁₅ est choisi parmi les radicaux alkyles en C₁-C₆ et les radicaux hydroxyalkyles
ou dihydroxyalkyles en C₁-C₆ ;
- R₁₆ est choisi parmi :
   - le radical R₁₉-CO- ;
   - les radicaux R₂₀ hydrocarbonés en C₁-C₂₂ linéaires ou ramifiés, saturés ou insaturés ;
   - l'atome d'hydrogène ;
- R₁₈ est choisi parmi :
   - le radical R₂₁-CO- ;
   - les radicaux R₂₂ hydrocarbonés en C₁-C₆ linéaires ou ramifiés, saturés ou insaturés ;
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ; sous réserve que la somme x + y + z vaut de 1 à 15 , que lorsque x vaut 0 alors R16 désigne R₂₀ et que lorsque z vaut 0 alors R₁₈ désigne R₂₂.

Les radicaux alkyles R₁₅ peuvent être linéaires ou ramifiés et plus particulièrement linéaires.
De préférence R₁₅ désigne un radical méthyle, éthyle, hydroxyéthyle ou dihydroxypropyle et plus particulièrement un radical méthyle ou éthyle.
Avantageusement, la somme x + y + z vaut de 1 à 10.
Lorsque R₁₆ est un radical R₂₀ hydrocarboné, il peut être long et avoir de 12 à 22 atomes de carbone ou court et avoir de 1 à 3 atomes de carbone.
Lorsque R₁₈ est un radical R₂₂ hydrocarboné, il a de préférence 1 à 3 atomes de carbone.
Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.
De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.
L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.
On utilise plus particulièrement les sels d'ammonium de formule (XIV) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical R₁₉-CO- ;
   - les radicaux méthyle, éthyle ou hydrocarbonés en C₁₄-C₂₂ ;
   - l'atome d'hydrogène ;
- R₁₈ est choisi parmi :
   - le radical R₂₁-CO- ;
   - l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés:
Avantageusement, les radicaux hydrocarbonés sont linéaires.

On peut citer par exemple les composés de formule (XIV) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Les lipides amphiphiles non-ioniques (A) formant la membrane des vésicules suivant l'invention sont de préférence choisis dans le groupe formé par :
- les esters et/ou les éthers de polyol et d'acide gras, polyoxyéthylénés ou non ;
- les esters et/ou les éthers d'acide gras d'α-butylglycoside ;
- les phospholipides synthétiques ou naturels, hydrogénés ou non.

Les esters ou les éthers de polyol et d'acide gras sont de préférence choisis parmi les mélanges d'esters ou les mélanges d'éthers d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités oxyde d'éthylène, et au moins un acide gras comportant une chaîne alkyle en C₅-C₂₂ , saturée ou non saturée, linéaire ou ramifiée, le nombre de chaînes alkyle par groupe polyol allant de 1 à 10.

Les esters de polyol et d'acides gras en C₅-C₂₂ particulièrement préférés sont ceux répondant à la formule (XV) suivante : 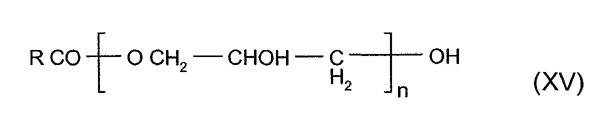 où n est une valeur statistique et qui peut contenir des proportions diverses d'esters pour lesquels n = 1, n = 2, n = 3, n = 4, etc ; c'est aussi le cas des esters comportant plusieurs chaînes alkyle dans leur partie lipophile, tels que les cocoates, qui contiennent des chaînes alkyles en C₅-C₂₂ ou les isostéarates où les chaînes alkyle sont en C₁₇ sont un mélange complexe de formes isomères ; c'est également le cas des produits constitués par des mélanges de mono-, di-, tri- ou polyesters d'un même polyol.

Parmi les produits commerciaux utilisables selon l'invention et ayant la structure d'un mélange d'esters de polyol et d'acide gras en C₅-C₂₂ tel que défini ci-dessus, on peut citer:
- les esters partiels de sorbitane (ou anhydride de sorbitol) et d'acide gras, vendus sous les dénominations commerciales "SPAN 20, 40, 60 et 80" par la Société "ICI" ;
- l'isostéarate de sorbitane, vendu sous la dénomination commerciale "SI 10 R NIKKOL" par la Société "NIKKO" ;
- le stéarate de sorbitane portant 4 unités oxyde d'éthylène, vendu sous la dénomination "TWEEN 61" par la Société "ICI" ;
- le stéarate de polyéthylèneglycol à 8 unités oxyde d'éthylène vendu sous le nom "MYR J 45" par "ICI" ;
- le monostéarate du polyéthylène glycol de formule (XVI) suivante:

   HOCH₂-(CH₂OCH₂)ₙCH₂OH (XVI)

   dans laquelle n est égal à 4, vendu sous la dénomination "MYS 4" par la Société "NIKKO" ;
- le stéarate de polyéthylèneglycol de poids moléculaire 400, qualité chimique ou qualité produite par biotechnologie, vendu par la Société "UNICHEMA" ;
- le stéarate de diglycéryle portant 4 unités d'oxyde d'éthylène, vendu sous la dénomination "HOSTACERINE DGS" par la Société "HOESCHT" ;
- le stéarate de tétraglycérol, vendu sous la dénomination "TETRAGLYN 1S" par la Société "NIKKO";
- l'isostéarate de diglycéryle, vendu par la Société "SOLVAY" ;
- le distéarate de diglycéryle, vendu sous la dénomination "EMALEX DSG 2" par la Société "NIHON" ;
- les mono-, di- et tri-palmitostéarate de sucrose, vendu sous les dénominations "F50, F70, F110 et F160 CRODESTA" par la Société "CRODA" ;
- le mélange de mono- et di-palmitostéarate de sucrose, vendu sous la dénomination "GRILLOTEN PSE 141 G" par la Société "GRILLO" ;
- le mélange de stéarate de sucrose et de cocoate de sucrose, vendu sous la dénomination "ARLATONE 2121" par la Société "ICI" ;
- le distéarate de méthylglucose portant 20 unités oxyde d'éthylène, vendu sous la dénomination "GLUCAM E20 DISTEARATE" par la Société "AMERCHOL".

Les esters et les éthers d'acide gras d'α-butylglucoside utilisés selon l'invention sont de préférence, soit des mélanges d'esters et/ou des mélanges d'éthers de différents acides gras d'α-butylglucoside dont les différentes chaînes grasses comportent, l'une par rapport à l'autre, un nombre d'atomes de carbone voisin (par exemple différent de 1 ou 2) soit des mélanges de mono-, di- tri- ou polyesters et/ou des mélanges de mono-, di- tri-polyéthers d'un même acide gras d'α-butylglucoside.

Les esters et les éthers d'acide gras d'α-butylglucoside utilisé(s) selon l'invention comporte(nt) de préférence une chaîne grasse ayant de 8 à 24 atomes de carbone, plus préférentiellement de 12 à 22 atomes de carbone et plus particulièrement de 14 à 18 atomes de carbone.

On peut citer par exemple, les esters et les éthers d'acide laurique (C₁₂), myristique (C₁₄), palmitique (C₁₆), stéarique (C₁₈), béhénique (C₂₂) d'α-butylglucoside.

On utilise plus particulièrement un mélange de mono- et de diester d'acide palmitique d'α-butylglucoside obtenu selon le procédé de fabrication enzymatique décrit dans les vésicules lipidiques conformes à l'invention.

Les esters et les éthers d'acide gras d'α-butylglucoside conformes à l'invention peuvent être préparés à partir d'α-butylglucoside obtenu selon le procédé de fabrication enzymatique décrit dans la demande de brevet FR-A-2680373 qui consiste à mettre en contact le butanol avec de l'amidon, des maltodextrines ou du maltose en présence d'une préparation enzymatique purifiée présentant une activité d'α-transglucosylation. Les esters et les éthers d'acide gras d'α-butylglucoside peuvent être synthétisés en faisant réagir l'acide gras ou le mélange d'acide gras correspondants avec l'α-butylglucoside selon des procédés classiques.

Les phospholipides synthétiques ou naturels, hydrogénés ou non préférés suivant l'invention sont choisis parmi la lécithine, de préférence hydrogénée, associé soit à du cholestérol et éventuellement à un tensioactif ionique, et un phytostérol oxyéthyléné comprenant de 2 à 50 motifs oxyde d'éthylène

A cette liste, il convient également d'ajouter les lipides amphiphiles décrits dans les documents FR 2 315 991 et FR 2 485 921.

De façon connue, on peut utiliser pour la fabrication des dispersions vésiculaires suivant l'invention des mélanges de lipides amphiphiles ioniques, des mélanges de lipides amphiphiles non ioniques et des mélanges de ces deux types de lipides.

Selon un mode préférentiel de l'invention, le rapport en poids entre la quantité de lipide amphiphile non-ionique (A) et de lipide amphiphile ionique (B) va de 50/1 à 50/25.

On peut, de façon connue, incorporer dans la phase lipidique constituant la membrane lipidique des vésicules à coeur aqueux de l'invention, au moins un additif qui a pour fonction principale de diminuer la perméabilité des vésicules, de prévenir leur floculation et leur fusion et d'augmenter le taux d'encapsulation.

Selon un mode préférentiel de l'invention, on peut ajouter à la phase lipidique au moins un additif choisi, de préférence, dans le groupe formé par :
- les stérols et notamment les phytostérols et le cholestérol,
- les alcools et diols à longue chaîne,
- les amines à longue chaîne et leurs dérivés ammonium quaternaire.
Ces additifs peuvent éventuellement avoir une activité cosmétique et/ou dermopharmaceutique. C'est, par exemple, le cas du cholestérol.
Leur taux va de 0 à 50% par rapport au poids total de la composition lipidique constituant les vésicules à coeur aqueux.

Dans la composition selon l'invention, les vésicules à coeur aqueux ont de préférence un diamètre moyen allant de 10 à 5 000 nm.

Avantageusement, la dispersion vésiculaire suivant l'invention peut également contenir au moins un autre céramide choisi parmi les céramides STAR, 1, 2, 2.5, 3, 4, 5 et/ou 6 (tels que décrits dans le document "The Normal Human stratum corneum: a new ceramide profile" M. Chopart *et al.* Prospectives in Percutaneous Penetration, 8th Internationak Conference Antibes Juan-Les Pins, France April 2-6, 2002), avantageusement les céramides STAR et/ou 4.
Le taux de céramide additionnel va de 0.001% à 30%, préférentiellement 0.001 à 10% et plus particulièrement 0.001% à 5% par rapport au poids total de la composition lipidique constituant les vésicules.

Par céramide 2.5 on entend un composé dont la constitution peut être représentée par la formule (IV) suivante : 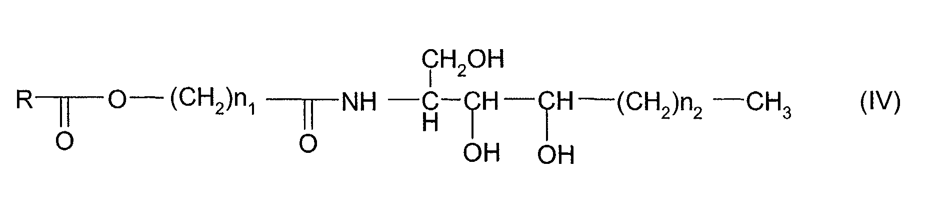 dans laquelle n₁ va de 22 à 35, de préférence de 25 à 33, de préférence encore de 26 à 29 et n₂ va de 11 à 21, de préférence de 13 à 17, de préférence encore de 13 à 15, et RCO désigne un résidu linoléoyle. La valeur de n₁ est de préférence égale à 29.

Les composés de formule (IV) peuvent être isolés à partir de prélèvements lipidiques réalisés par des méthodes non invasives sur des volontaires sains. Ces prélèvements lipidiques sont ensuite soumis à un traitement préparatif et analytique permettant de séparer et d'identifier les familles céramidiques.

Une autre source potentielle des composés de formule (IV) réside dans l'utilisation d'enzymes telles que des trans-acylases agissant sur des précurseurs ou des modulateurs de ces enzymes.

Les précurseurs sont notamment des composés de formule (V) suivante: 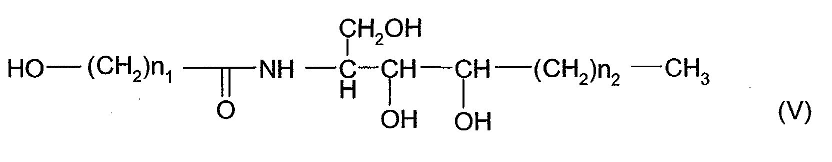 dans laquelle n₁ va de 22 à 35, de préférence entre 25 et 33, de préférence encore entre 26 et 29 et n₂ va de 11 à 21, de préférence de 13 à 17, de préférence encore de 13 à 15. La valeur de n₁ est de préférence égale à 29.

Ces précurseurs correspondent aux composés de formule (IV) non estérifiés sur l'OH terminal de l'acide gras hydroxylé.

Par céramide STAR on entend un composé dont la constitution peut être représentée par la formule (VI) suivante : 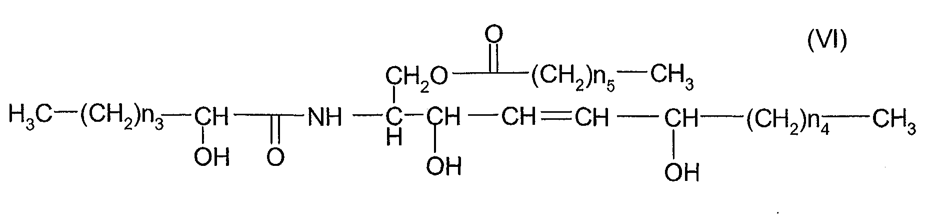 dans laquelle n₃ est un nombre entier allant de 17 à 35, de préférence de 21 à 30, préférentiellement de 23 à 28, de préférence encore est égal à 25, 26 ou 27, n₄ est un nombre entier allant de 9 à 18, de préférence de 11 à 15, préférentiellement de 11 à 13, de préférence encore égal à 11, et n₅ est un nombre entier allant de 12 à 18, de préférence de 14 à 16, de préférence égal à 14.

Les composés de formule (VI) peuvent être isolés à partir de prélèvements lipidiques réalisés par des méthodes non invasives sur des volontaires sains. Ces prélèvements lipidiques sont ensuite soumis à un traitement préparatif et analytique permettant de séparer et d'identifier les familles céramidiques.

Une autre source potentielle des composés formule (VI) réside dans l'utilisation d'enzymes telles que des trans-acylases agissant sur des précurseurs ou des modulateurs de ces enzymes.

Les précurseurs sont notamment des composés de formule (VII) suivante: 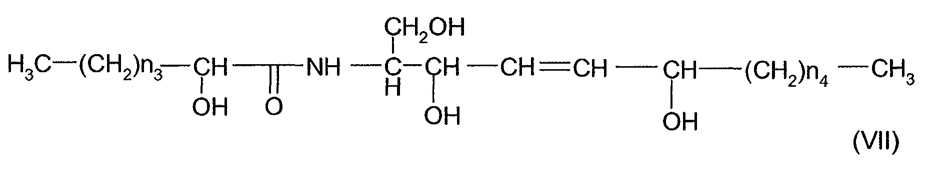 dans laquelle n₃ est un nombre entier allant de 17 à 35, de préférence de 21 à 30, préférentiellement de 23 à 28, de préférence encore de 25 à 27, n4 est un nombre entier allant de 9 et 18, de préférence de 11 à 15, , préférentiellement de 11 à 13, de préférence encore égale à 11.
Ces précurseurs correspondent aux composés de formule (VI) non estérifiés en position 1.

Avantageusement, la dispersion vésiculaire suivant l'invention peut également contenir au moins un actif additionnel.
Si les actifs sont hydrosolubles, on les introduit dans la phase hydrophile encapsulée des vésicules.
Si les actifs sont liposolubles, on les introduit dans la phase lipidique constituant la membrane.
Si les actifs sont amphiphiles, ils se répartissent entre la phase lipidique et la phase hydrophile encapsulée avec un coefficient de partage, qui varie selon la nature de l'actif amphiphile et les compositions respectives de la phase lipidique et de la phase hydrophile encapsulée.

Comme actifs, on peut utiliser au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents tenseurs ; les agents anti-pollution et/ou anti-radicalaire ; les agents agissant sur la microcirculation ; les agents agissant sur le métabolisme énergétique des cellules ; et leurs mélanges.

Avantageusement, la dispersion vésiculaire suivant l'invention peut également contenir au moins un autre composé permettant d'améliorer la fonction barrière. Ce dérivé est choisi parmi l'acide ascorbique (vitamine C) ou ses analogues, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline.
De préférence, le composé permettant d'améliorer la fonction barrière est l'acide ascorbique sous forme D ou L, avantageusement sous forme L ou ses analogues choisis parmi ses sels, de préférence l'ascorbate de sodium, l'ascorbylphosphate de magnésium ou de sodium, ses esters, de préférence ses esters acétique, propionique ou palmitique, ou ses sucres, de préférence l'acide ascorbique glycosylé.

De façon connue, la composition selon l'invention peut contenir également des adjuvants habituels dans le domaine cosmétique, tels que les conservateurs, les antioxydants, les solvants, les parfums, les absorbeurs d'odeur, les neutralisants, les filtres solaires, les polymères, les émulsionnants et les coémulsionnants, et les matières colorantes.

La composition suivant l'invention peut être introduite dans tout support cosmétique se présentant sous toutes les formes galéniques classiquement utilisées dans le domaine cosmétique: il peut s'agir notamment d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H). Ces formes sont préparées selon les méthodes usuelles. On préfère utiliser selon cette invention un support cosmétique sous la forme d'une émulsion huile-dans-eau.

L'invention est illustrée plus en détail dans les exemples suivants. Ces exemples ne sauraient limiter en aucune façon la portée de l'invention.

### EXEMPLE 1 : Préparation du céramide 7 de formule (I) par CCM à partir de stratum corneum humain

### Etape 1: Extraction par turbine

Echantillon de départ : Epiderme d'un volontaire sain
Action : passage de solvants organiques (le plus souvent un mélange d'hexane/éthanol 2/3) au travers du stratum corneum pour entraîner les lipides grâce à une turbine.
Echantillon final : Lipides épidermiques sous forme solvatée

### Etape 1 bis: Extraction à partir de stripping vernis

Echantillon de départ : Epiderme d'un volontaire sain
Action : application d'une bande adhésive -vernis+nylon- sur la peau de volontaires, puis arrachage de l'ensemble entraînant par la même une partie du stratum. Les strips sont ensuite mis en présence de solvants (de type Chloroforme Méthanol (2/1) sous agitation.
Echantillon final : Lipides épidermiques sous forme solvatée

### Etape 2: Isolement des céramides 7

Echantillon départ : Lipides épidermiques sous forme solvatée (lipides totaux)
Action : Séparation des familles céramidiques
- Après concentration si nécessaire du pool lipidique ainsi obtenu (la concentration étant obtenue par évaporation d'une partie des solvants) , le pool de lipides est déposé sur une plaque de silice Whatman LK5 de 20x20 cm ou 5721 Merk et on procède à 2 élutions successives avec un mélange chloroforme /méthanol /acide acétique dans les rapports 190/5/1 pour la première élution et 190/9/1 pour la seconde élution.
- Les classes de céramides sont repérées en ultraviolet (à 254 nm) après vaporisation sur la plaque d'une solution de primuline à 5 mg/100 ml (révélation de la laque). Cette observation permet de délimiter une dizaine de zones contiguës sur la plaque de silice numérotées par exemple de 1 à 10 en allant de la zone la plus éluée à la zone la moins éluée. La silice de chacune de ces zones est grattée, récupérée et extraite (pour en extraire les céramides) à plusieurs reprises avec un mélange de chloroforme /méthanol (2/1). Les phases organiques sont rassemblées et lavées à l'eau puis évaporées à sec pour obtenir les composés céramidiques purs. Ces céramides (regroupés donc par bandes de migration) sont alors remis en solution dans un mélange chloroforme/méthanol (2/1 ). Une faible proportion de chaque échantillon est prélevée pour identification analytique (voir analyse structurale des composés céramidiques paragraphe suivant) du contenu en céramides afin de déterminer la classe du céramide majoritaire et quelles sont les impuretés (autres céramides généralement). Il est ainsi possible d'identifier l'échantillon contenant le céramide 7, généralement mélangé avec du céramide 6 (impureté)
Echantillon final : Céramide 7 (+ Céramide 6 sous forme d'impuretés) solvaté

### Etape 3: Purification de l'échantillon de céramide 7 si nécessaire

Echantillon départ : Céramide 7 (+ Céramide 6 sous forme d'impuretés) solvaté
Action : L'échantillon contenant le céramide 7 est alors purifié en répétant l'étape 2 : dépôt de l'échantillon, révélation, grattage de la silice et extraction des céramides, identification de la bande comprenant principalement le céramide 7 (analyse structurale finale).
Echantillon final : Céramide 7 de formule (I) purifié

### Analyse structurale des composés céramidiques :

L'échantillon destiné à l'analyse est réparti en deux fractions.

On réalise une dérivation de la première fraction céramides au moyen du chlorure de benzoyle. Les dérivés benzoylés ainsi obtenus sont séparés en chromatographie liquide haute performance et injectés dans un spectrographe de masse en sortie de colonne par couplage HPLC-MS.

La deuxième fraction subit une hydrolyse alcaline pour libérer les bases sphingoïdes contenues dans les céramides. Les bases libérées sont dérivées à l'orthophtalaldéhyde avant d'être séparées en HPLC avec détection en fluorescence.
L'ensemble des résultats analytiques ainsi obtenu permet d'attribuer une structure moléculaire précise à chaque céramide présent dans l'échantillon.

### EXEMPLE 2: Préparation du céramide 5.5 par CCM à partir de stratum corneum humain

La préparation du céramide 5.5 est identique à celle du céramide 7 de l'exemple 1 à la différence que la bande isolée après analyse structurale du contenu en céramides des différentes bandes de migration sera la bande contenant principalement du céramide 5.5, et à la différence que ce dernier est généralement associé à une plus grande quantité d'impuretés (céramides 5) et que son obtention sous une forme correctement purifiée nécessite éventuellement de renouveler une fois ou plus si nécessaire l'étape 3 de purification.

### EXEMPLE 3 Evaluation de l'efficacité des dispersions vésiculaires suivant l'invention sur la fonction barrière des peaux reconstruites

On prépare les deux suspensions suivantes:

| Formule aux Niosomes sans céramide 7 | |
|---|---|
| Palmitate de sorbitan ( Span 40 commercialisé par Uniqema) | 0.225% |
| Cholestérol | 0.225% |
| N Stéaroyl L Glutamique acide disodique | 0.050% |
| Propylène glycol | 3.000% |
| Eau | qsp 100.000% |

| Formule aux niosomes contenant du céramide 7 | |
|---|---|
| Palmitate de sorbitane | 0.2250% |
| Cholesterol | 0.1250% |
| N stearoyl I glutamic acid disodium | 0.0500% |
| Céramide 7 | 0.0315% |
| Eau distillée | qsp 100.0000% |

### Mode opératoire

Dans les 2 cas, les lipides sont associés préalablement en phase solvant méthanol/chloroforme (50/50 en poids). Le solvant est ensuite évaporé sous pression réduite à l'aide d'un évaporateur rotatif. Le film lipidique formé sur la paroi du ballon est alors solubilisé par une solution aqueuse d'octylglucoside (6% ) pour former des micelles mixtes Octylglucoside/Lipides vésiculaires. Cette solution est ensuite dialysée pendant 24h contre de l'eau distillée. Les liposomes se forment au fur et à mesure que l'octylglucoside est dialysé.

Une étude est alors réalisée sur trois kits de douze puits contenant 1 cm² d'épiderme EPISKIN^{TM} chacun.
Les différents traitements sont :
- un kit témoin c'est à dire non traité
- un kit placébo c'est à dire traité par une formule aux niosomes sans céramide 7
- un kit traité par le céramide 7 formulé au sein d'une formule aux niosomes (formule CER 7)

Les trois kits sont traités en topique : pour chaque type de traitement (à l'exception du kit témoin), deux microlitres de formule sont appliqués à la surface de chaque épiderme reconstruit. Le traitement est renouvelé deux fois à 48 H d'intervalle. Les épidermes sont ensuite prélevés, les surfaces lavées, et les lipides contenus dans chaque épiderme extraits. Les sphingolipides (céramides essentiellement) sont enfin quantifiés par analyse de leur base sphingoide.

Dans les conditions de traitements présentées ci dessus, les résultats montrent une augmentation sensible des bases 6-hydroxy-sphingénines retrouvées dans l'épiderme des modèles de peaux reconstruites EPISKIN™ traité par la formule aux liposomes contenant du céramide 7 par rapport à un épiderme non traité ou traité par le placebo comme le montre l'histogramme de la Figure 2.
La formulation du céramide 7 au sein d'une dispersion vésiculaire le stabilisant et le rendant biodisponible permet d'améliorer le profil céramidique des épidermes reconstruits du modèle EPISKIN™. Or, l'augmentation de la proportion de céramides à base 6-hydroxy-sphingénine grâce à l'augmentation de la proportion de céramide 7 dans le profil des modèles de peau reconstruite comme EPISKIN™ permettent une augmentation de la fonction barrière du modèle.
Ce procédé permet donc, en rendant la fonction barrière des modèles de peau reconstruite plus proche de la fonction barrière de la peau humaine normale, d'améliorer celle-ci.

### EXEMPLE 4 : Effet du céramide 7 formulé dans un niosome suivant l'invention sur la fonction barrière d'épidermes reconstruits EPISKIN™

Principe de la méthode d'une étude de pénétration: Evaluer l'état de la fonction barrière des épidermes reconstruits du modèle EPISKINT™ traités avec le céramide 7 dans une formule niosomes par voie topique, par une étude de pénétration de la caféine radio marquée sur cellules de diffusion en mode statique : la caféine radiomarquée, molécule amphiphile, pénètre plus ou moins à travers l'épiderme selon la qualité de celui ci ; plus la molécule passe à travers, plus la fonction barrière est altérée.

Les traitements des épidermes reconstruits du modèle EPISKIN^{TM} sont effectués entre le 8° et le 14° jour de culture par application à la surface des épidermes de 2 µl soit de la formule niosomes contenant le céramide 7, soit de la formule sans céramide 7 (appelé formule placébo ) comme proposées dans l'exemple 3.

Au 14 ° jour de culture et après stabilisation des modèles dans les cellules de diffusion, 90µL (322µL/cm²) de solution de caféine C¹⁴ en excès (10mM en glycérol, 135µCi/mMol) sont appliqués en surface des épidermes. Après 16h00 de pénétration, l'excès est prélevé et récupéré. La peau est récupérée à son tour, les tissus digérés. La radioactivité du milieu, puis de la peau, puis de l'excès sont alors comptabilisés au compteur à scintillation.

Les quantités totales de radioactivité ainsi récupérées sont ensuite ramenées à une même valeur expérimentale de dépôt afin de normaliser ces derniers et ainsi pouvoir mieux comparer l'effet des différents traitements. De plus, Pour avoir un résultat le plus représentatif possible de l'étude, les valeurs suivantes ont été couplées : Excès de surface + lavages (car les lavages constituent tout ce qui se trouve au dessus de l'épiderme) d'une part et Milieu récepteur + tissu (car tout ce qui pourrait être retrouvé dans le tissu a donc pénétré à travers).

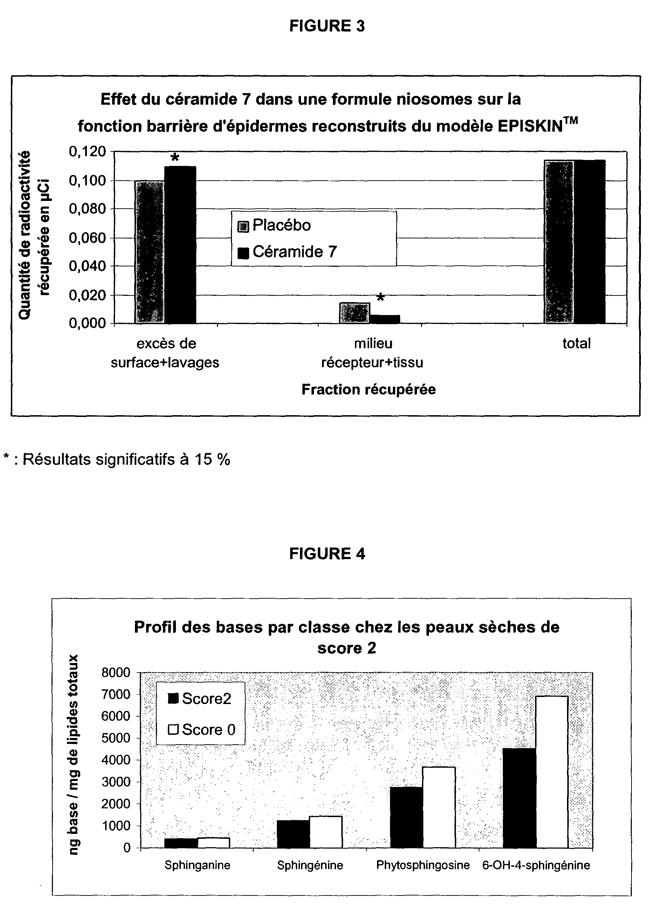

Les résultats de cette étude sont présentés sur l'histogramme de la figure 3.
L'histogramme de la figure 3 montre une fonction barrière améliorée pour les peaux reconstruites du modèle EPISKINT™ après traitement par du céramide 7 formulé dans des niosômes puisque moins de caféine a pénétré dans les tissus et plus est restée en surface.

### EXEMPLE 5: Mise en évidence du déficit en céramides à bases 6-hydroxy-4-sphingénine chez les peaux sèches.

Dans cette étude, les profils lipidiques d'échantillons de peaux normales (score 0 de sécheresse) et sèches (score 2 de sécheresse) ont été comparés. Les prélèvements (lipides directement extraits à l'aide d'une turbine par des solvants traversant le stratum) sont effectués sur 22 sujets de score 0 et 19 sujets de score 2. Les échantillons ont ensuite été poolés par score pour des raisons de sensibilité à l'analyse.

Les céramides ont donc été identifiés par HPLC / détection spectrométrie de masse après séparation des différentes classes par chromatographie sur couche mince. Une quantification des céramides contenus dans chaque bande de migration a ensuite été effectuée par HPLC /détection fluorimétrique après hydrolyse des céramides en bases et acides gras et dérivation spécifique des bases (approche quantitative très précise).

Les résultats sont présentés sur la figure 4
Cette étude montre donc que la quantité moyenne de céramides à bases 6 hydroxy-4-sphingénine (CER 5.5, 7 et 4) est inférieure chez les peaux sèches de score 2 par rapport aux peaux normales de score 0.

### EXEMPLE 6: Préparation du céramide STAR

### Prélèvement lipidique :

Les prélèvements ont lieu sur une cohorte de 22 femmes (volontaires sains) d'âge moyen 33,8 ans +/- 8,8 et présentant une peau qualifiée de normale par les cliniciens. Les prélèvements sont réalisés sur l'avant bras à l'aide d'une turbine après nettoyage de la peau au moyen d'un coton imbibé d'éther afin d'éliminer les traces de sébum. La chambre d'extraction de la turbine, remplie avec 10 ml de mélange hexane/éthanol (2/3) est appliquée sur une surface de 12,56 cm². Le mélange est agité durant 1 minute puis est collecté à l'aide d'une seringue en verre puis stocké dans un récipient en verre à -20 °C.
Trois prélèvements sont ainsi réalisés sur l'avant bras de chaque personne. Les échantillons sont ensuite rassemblés, évaporés à sec à l'aide d'un évaporateur rotatif, repris dans 1 ml de chloroforme /méthanol (2/1) et conservé à -20 °C. L'extrait sec correspondant à l'ensemble de ces prélèvements est de 36,4 mg et représente une surface extraite de 829 cm².

### Séparation et isolement des céramides :

Dans un premier temps, on sépare les céramides des autres catégories de lipides en déposant l'échantillon lipidique précédemment obtenu sur une cartouche de silice phase normale (gel de silice 60). Après élimination des lipides neutres par 10 ml de chloroforme contenant 1% d'acide acétique, les céramides sont élués avec 10 ml de mélange chloroforme /méthanol (95/5). Les céramides sont ensuite stockés dans 1 ml de chloroforme à -20 °C.

Dans un second temps, les différents céramides sont séparées par chromatographie couche mince dans les conditions suivantes :
On dépose 1,7 mg du mélange de céramides obtenus à l'issue de la première étape sur une plaque de silice Whatman LK5 de 20x20 cm et on procède à 2 élutions successives avec un mélange chloroforme /méthanol /acide acétique dans les rapports 190/5/1 pour la première élution et 190/9/1 pour la seconde élution.

Les classes de céramides sont repérées en ultraviolet (à 254 nm) après vaporisation sur la plaque d'une solution de primuline à 5 mg/100 ml. Cette observation permet de délimiter 10 zones contiguës sur la plaque de silice numérotées de 1 à 10 en allant de la zone la plus éluée à la zone la moins éluée. La silice de chacune de ces zones est grattée, récupérée et extraite à plusieurs reprises avec un mélange de chloroforme /méthanol (2/1). Les phases organiques sont rassemblées et lavées à l'eau puis évaporées à sec pour obtenir les composés céramidiques purs. Une partie de ces céramides est ensuite remise en solution dans un mélange chloroforme/méthanol (2/1 ) pour identification analytique.

On récupère dans la tache no1 de la plaque de silice, les céramides décrits précédemment tels que n₃ va de 17 à 35, n₄ est va de 9 à 18, et n₅ va de 12 à 18.

### Analyse structurale des composés céramidiques :

L'échantillon destiné à l'analyse est réparti en deux fractions.

On réalise une dérivation de la première fraction céramides au moyen du chlorure de benzoyle. Les dérivés benzoylés ainsi obtenus sont séparés en chromatographie liquide haute performance et injectés dans un spectrographe de masse en sortie de colonne par couplage HPLC-MS.

La deuxième fraction subit une hydrolyse alcaline pour libérer les bases sphingoïdes contenues dans les céramides. Les bases libérées sont dérivées à l'orthophtalaldéhyde avant d'être séparées en HPLC avec détection en fluorescence.

L'ensemble des résultats analytiques ainsi obtenu permet d'attribuer une structure moléculaire précise à chaque céramide présent dans l'échantillon.

Dans le mélange, on isole une fraction A contenant les céramides tels que n₃ va de 25 et 27, n₄ est égal à 11, et n₅ est égal à 14.

### EXEMPLE 7 : Préparation du céramide 2.5

La préparation du céramide 2.5 est identique à celle du céramide STAR de l'exemple 6 à la différence de l'étape de séparation et d'isolement des céramides à l'issu de laquelle on récupère dans la tache n°4 de la plaque de silice, les céramides décrits précédemment tels que n₁ va de 22 à 35 et n₂ va de 11 à 21 et RCO désigne un résidu linoléoyle. En outre, au cours de l'analyse structurale des composés céramidiques on isole dans le mélange, une fraction A contenant les céramides telles que n₁ est égal à 29, et n₂ va de 13 à 15.

### EXEMPLE 8 : Milieu de culture sur lequel on peut additionner un dérivé de la famille du céramide 7 en association avec le BSA

- Milieux de cultures DMEM (3 volumes) et HAM F12 (1 volume) commercialisés par DUBELCCO et GIBCO 450 ml
- Sérum de veau supplémenté en fer 50 ml
- EGF (facteur de croissance épidermique). (10 ng / ml) 500 µl
- Isoprotérénol (10-⁶M) 500 µL
- Hydrocortisone (0,4 µg/ml) 400 µl
- L-Glutamine (2mM) 5 ml
- D.L α Tocophérol acétate 10.6 µmoles
- L Carnitine 5.1 µmoles
- BSA (Bovine Serum Albumine) 12.1 µmoles
- Céramide 7 175 µg
   (0.25 µmoles)

### EXEMPLE 9 : Compositions

### Formule aux Liposomes contenant le céramide 7

- Lécithine de soja (enrichie à 75% de phosphatidylcholine) vendue par SEPPIC sous la dénomination commerciale Lipoïd S75 0.5000%
- Propylène glycol 3.0000%
- Céramide 7 0.0315%
- Eau qsp 100.0000%

### Formule aux Liposomes contenant le céramide 5.5

Lécithine de soja (enrichie à 75% de PC) Lipoïd S75 0,5000% Propylène glycol 3,0000% Céramide 5.5 0,0315% Eau qsp 100%

### Creme hydratante à base d'une suspension de Ceramide 7

### Phase vésiculaire

- Palmitate de sorbitane 2,250%
- Cholesterol 1,125%
- N stearoyl I glutamic acid disodium 0,500%
- Céramide 7 0,315%
- Glycérine 5,000%
- Méthylparaben 0,300%
- Eau déminéralisée qsp 100%

### Phase A1:

- Stearyl heptanoate 4,0%
- Vaseline codex 1,5 %
- Huile d'avocat 3,2 %
- Huile de jojoba 3,0%
- Huile de silicone volatile 2,7 %
- Acétate de vitamine E 1,0%
- D α tocophérol naturel commercialisé par la Société Henkel sous le nom « COPHEROL 1300 » 1 %
- Glycérides de Vitamine F 3%

### Phase A2 :

- Gomme de silicone commercialisée par Dow Corning sous le nom « Q2-1403 FLUID » 3,0%
- Propylparaben 0,2%
- Parfum 0,3%

### Phase B :

- Mélange de polymères carboxyvinyliques commercialisé sous le nom « CARBOPOL 940 » par la Société Goodrich 0,40%
- Eau déminéralisée 9,50%
- Triéthanolamine 0,25%

La phase vésiculaire est préparée selon l'un des procédés déjà décrits puis homogénéisée par 2 passages par un homogénéisateur haute pression, à 500b, de type Soavi OBL 20 ou Microfluidics.
Les phases A1 et A2 sont ensuite ajoutée sous agitation rotor-stator suffisante pour faire une prédispersion d'huile suffisamment stable. L'ensemble est ensuite homogénéisé 2 fois à 500b.
La phase B, préalablement préparée, est ajoutée à l'émulsion précédemment préparée et dispersée à l'aide d'une turbine type défloculeuse.
On obtient une crème blanche, lisse, adaptée au traitement des peaux sèches.

## Revendications

1. Procédé de préparation d'un épiderme reconstruit ou d'un équivalent de peau supplémenté en au moins un dérivé du céramide 7 et/ou 5.5 de formule (I), comprenant l'introduction d'au moins un dérivé du céramide 7 et/ou 5.5 dans le milieu de culture dudit épiderme reconstruit ou dudit équivalent de peau et/ou l'application topique sur la surface dudit épiderme reconstruit ou dudit équivalent de peau d'une composition à base de vésicules lamellaires lipidiques incorporant au moins un dérivé de la famille du céramide 7 et/ou 5.5 de formule (I) suivante: 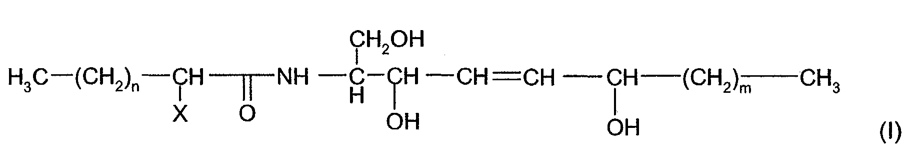 dans laquelle :
- X représente un atome d'hydrogène ou un groupement hydroxyle (OH), avantageusement un groupement hydroxyle ;
- n représente un nombre entier allant de 19 à 29, de préférence de 21 à 27, avantageusement égal à 21, 22 ou 23 ;
- m représente un nombre entier allant de 9 à 19, de préférence de 9 à 15, avantageusement égal à 11, 12 ou 13.

2. Procédé suivant la revendication 1, **caractérisé en ce que** n va de 21 à 27; m va de 9 et 15.

3. Procédé suivant l'une quelconque des revendications précédentes, **caractérisé en ce que** n est égal à 21, 22 ou 23; m est égal à 11, 12 ou 13.

4. Procédé suivant l'une quelconque des revendications précédentes, **caractérisé en ce que** n est égal à 21 et m est égal à 11.

5. Procédé suivant l'une quelconque des revendications précédentes, **caractérisé en ce que** X représente un groupement hydroxyle.

6. Procédé suivant l'une quelconque des revendications précédentes, **caractérisé en ce que** l'introduction dans le milieu de culture d'au moins un dérivé de céramide 5.5 et/ou 7 de formule (I) est réalisée après dissolution préalable dudit dérivé ou de la dite composition dans un solvant.

7. Procédé suivant la revendication 6, **caractérisé en ce que** le solvant est l'éthanol ou le DMSO étant entend que le rapport final en solvant introduit dans le milieu de culture ne doit pas dépasser 1/1000.

8. Procédé suivant l'une quelconque des revendications 1 à 6, **caractérisé en ce que** l'on introduit dans le milieu de culture une association contenant au moins un dit dérivé de formule (I) et au moins une molécule capable de véhiculer et de rendre biodisponible le dit dérivé au sein de l'épiderme reconstruit ou dudit équivalent de peau à partir du milieu de culture.

9. Procédé suivant la revendication 8, **caractérisé en ce que** dans l'association la dite molécule est choisie parmi le BSA et/ou un composé de la famille des cyclodextrines, étant entendu que la concentration de BSA pouvant être introduite dans le milieu de culture doit être inférieure ou égale à 100 µmol/l.

10. Procédé suivant l'une quelconque des revendications 8 et 9, **caractérisé en ce que** l'association comprend en outre au moins un agent antioxydant et un transporteur cellulaire, étant entendu que la concentration en agent antioxydant et en transporteur cellulaire pouvant être introduit dans le milieu de culture doit être inférieure ou égale à 50 µmol/l et 100µmol/l respectivement de milieu de culture.

11. Procédé suivant la revendication précédente, **caractérisé en ce que** l'agent antioxydant est le D.L α Tocophérol acétate le transporteur cellulaire est la L Carnitine.

12. Procédé suivant l'une quelconque des revendications 1 à 6, **caractérisé en ce que** l'on introduit dans le milieu de culture une composition à base de vésicules lamellaires lipidiques incorporant au moins un dérivé de la famille du céramide 7 et/ou 5.5 de formule (I).

13. Procédé suivant l'une quelconque des revendications précédentes, **caractérisé en ce que** la concentration d'au moins un dérivé de formule (I) introduit dans le milieu de culture va de 10 g/l à 10⁻⁶ g/l.

14. Procédé suivant l'une quelconque des revendications 1 à 6, **caractérisé en ce que** l'application topique sur la surface dudit épiderme reconstruit ou dudit équivalent de peau est réalisée avec une composition à base de vésicules lamellaires lipidiques incorporant au moins un dérivé de la famille du céramide 7 et/ou 5.5 de formule (I).

15. Composition comprenant une dispersion, dans une phase aqueuse externe, de vésicules formées par des phases lamellaires lipidiques séparées les unes des autres par des couches hydrophiles, lesdites phases lamellaires comprenant au moins un lipide amphiphile, et au moins un dérivé de formule (I) telle que définie dans l'une quelconque des revendications 1 à 5 inclus dans lesdites phases lamellaires lipidiques.

16. Composition selon la revendication précédente, **caractérisée en ce que** les vésicules sont des niosomes ou des liposomes.

17. Composition selon l'une quelconque des revendications 15 à 16, **caractérisée en ce que** le taux de dérivé de formule (I) représente de 0.001% à 30% par rapport au poids total de la composition lipidique constituant les vésicules.

18. Composition selon l'une quelconque des revendications 15 à 17, **caractérisée en ce que** le taux de dérivé de formule (I) représente de 0.001% à 10%, de préférence 0.001% à 5% par rapport au poids total de la composition lipidique constituant les vésicules.

19. Composition selon l'une quelconque des revendications 15 à 18, **caractérisée en ce que** le rapport en poids entre la quantité de phase lipidique et la quantité de phase aqueuse de la dispersion allant de 1/1000 à 300/1000.

20. Composition selon l'une quelconque des revendications 15 à 19, **caractérisée en ce que** les phases lamellaires comprennent au moins un lipide amphiphile non ionique, choisi parmi :
- les esters et/ou les éthers de polyol et d'acide gras, polyoxyéthylénés ou non ;
- les esters et/ou les éthers d'acide gras d'α-butylglycoside;
- les phospholipides synthétiques ou naturels, hydrogénés ou non.

21. Composition suivant la revendication 20, **caractérisée en ce que** les esters ou les éthers de polyol et d'acide gras sont choisis parmi les mélanges d'esters ou les mélanges d'éthers d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités oxyde d'éthylène, et au moins un acide gras comportant une chaîne alkyle en C₅-C₂₂ , saturée ou non saturée, linéaire ou ramifiée, le nombre de chaînes alkyle par groupe polyol allant de 1 à 10.

22. Composition suivant la revendication 20, **caractérisée en ce que** les esters et/ou les éthers d'acide gras d'α-butylglycoside sont choisis parmi :
- des mélanges d'esters et/ou des mélanges d'éthers de différents acides gras d'α-butylglucoside dont les différentes chaînes grasses comportent, l'une par rapport à l'autre, un nombre d'atomes de carbone voisin (par exemple différent de 1 ou 2) ou,
- des mélanges de mono-, di- tri- ou polyesters et/ou des mélanges de mono-, di- tri-polyéthers d'un même acide gras d'α-butylglucoside;
les dits esters et lesdits éthers d'acide gras d'α-butylglucoside comportant une chaîne grasse ayant de 8 à 24 atomes de carbone.

23. Composition selon l'une quelconque des revendications 20 à 22, **caractérisée en ce que** les phases lamellaires comprennent en outre au moins un lipide amphiphile ionique.

24. Composition suivant selon la revendication 23, **caractérisée en ce que** le lipide amphiphile ionique est choisi dans le groupe formé par :
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides ;
- les dérivés alkylsulfoniques notamment de formule (X) : 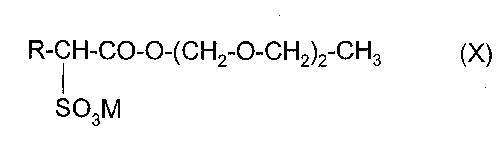 dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium ; et leurs mélanges;
- les sels d'ammonium quaternaire, les amines grasses et leurs sels.

25. Composition selon la revendication 24, **caractérisée en ce que** les sels d'ammonium quaternaire sont choisis parmi:
- Les sels d'ammonium quaternaires représentés par la formule (XI) suivante; 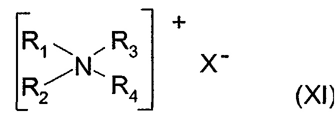 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle; X est un anion choisi dans le groupe des halogénures, phosphates, acétates, lactates, alkyl(C₂-C₆)sulfates, alkyl-ou-alkylarylsulfonates;
- les sels d'ammonium quaternaire de l'imidazolinium représentés par la formule (XII) suivante: 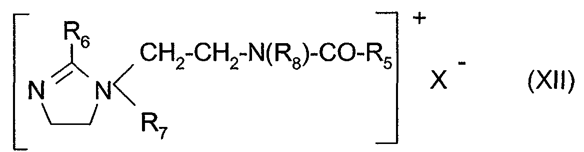 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates;
- les sels de diammonium quaternaire représentés par la formule (XIII) suivante; 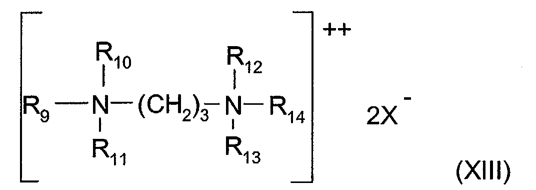 dans laquelle R₉ désigne un radical aliphatique comportant environ de 16 à 30 atomes de carbone, R₁₀, R_{11'} R_{12'} R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates;
- les sels d'ammonium quaternaire contenant au moins une fonction ester.

26. Composition selon la revendication 24, **caractérisée en ce que** les sels d'ammonium quaternaire contenant au moins une fonction ester répondent à la formule (XIV) suivante : 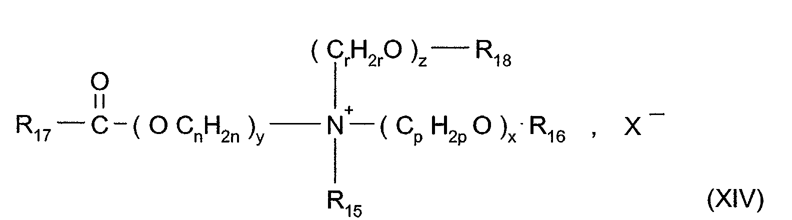 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
• le radical R₁₉-CO- ;
• les radicaux R₂₀ hydrocarbonés en C₁-C₂₂ linéaires ou ramifiés, saturés ou insaturés ;
• l'atome d'hydrogène ;
- R₁₈ est choisi parmi :
• l'atome d'hydrogène ;
• le radical R₂₁-CO- ;
• les radicaux R₂₂ hydrocarbonés en C₁-C₆ linéaires ou ramifiés, saturés ou insaturés ;
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

27. Composition selon la revendication 26, **caractérisée en ce que** les sels d'ammonium quaternaire contenant au moins une fonction ester correspond à la formule (XIV) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
• le radical R₁₉-CO- ;
• les radicaux méthyle, éthyle ou hydrocarbonés en C₁₄-C₂₂ ;
• l'atome d'hydrogène ;
- R₁₈ est choisi parmi :
• le radical R₂₁-CO- ;
• l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.

28. Composition selon l'une quelconque des revendications 20 à 27, **caractérisée en ce que**, le rapport en poids entre la quantité de lipide amphiphile non-ionique et de lipide amphiphile va de 50/1 à 50/25.

29. Composition selon l'une quelconque des revendications 15 à 28, **caractérisée en ce que** lesdites phases lamellaires contiennent au moins un additif choisi parmi les stérols, les alcools et diols à chaîne grasse, les amines à chaîne grasse et leurs dérivés ammonium quaternaire.

30. Composition selon la revendication 29, **caractérisée en ce que** ledit additif est le cholestérol.

31. Composition selon l'une quelconque des revendications 15 à 30, **caractérisée en ce que** la composition comprend au moins un autre céramide inclus dans lesdites phases lamellaires lipidiques, le dit céramide étant choisi parmi les céramides STAR, 1, 2, 2.5, 3, 4, 5 et/ou 6.

32. Composition suivant la revendication 31, **caractérisée en ce que** le céramide est choisi parmi les céramides STAR et/ou 4.

33. Composition suivant l'une quelconques des revendications 15 à 32, **caractérisée en ce que** la composition contient un autre composé permettant d'améliorer la fonction barrière choisi parmi l'acide ascorbique ou ses analogues, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline.

34. Composition suivant l'une quelconques des revendications 15 à 33, **caractérisée en ce qu'**elle contient un actif additionnel choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents tenseurs ; les agents anti-pollution et/ou anti-radicalaire ; les agents agissant sur la microcirculation ; les agents agissant sur le métabolisme énergétique des cellules ; et leurs mélanges.

35. Composition suivant l'une quelconques des revendications 15 à 34, **caractérisée en ce qu'**elle contient un adjuvant choisi parmi les conservateurs, les antioxydants, les solvants, les parfums, les absorbeurs d'odeur, les neutralisants, les filtres solaires, les polymères, les émulsionnants et les coémulsionnants, et les matières colorantes.

36. Utilisation topique d'une composition suivant l'une quelconque des revendications 15 à 35 pour :
- renforcer la fonction barrière des épidermes humains normaux, et/ou
- améliorer la fonction barrière d'un épiderme présentant un déficit en céramides à base 6-hydroxy-4-sphingénine, de préférence des peaux sèches, des peaux rugueuses et/ou abîmées et/ou âgées et/ou sensibles, et/ou
- rétablir ou maintenir l'intégrité du stratum corneum, et/ou
- améliorer l'aspect de surface et/ou l'hydratation de la peau, et/ou
- améliorer et/ou maintenir le contenu lipidique de l'épiderme humain.

37. Utilisation d'une composition suivant l'une quelconque des revendications 15 à 35 pour la fabrication d'une composition destinée au traitement des peaux atopiques.

38. Procédé de traitement cosmétique d'embellissement ou d'hydratation de la peau humaine, caractérisé à ce qu'il consiste à appliquer topiquement sur la peau une composition suivant l'une quelconque des revendications 15 à 35.

39. Procédé suivant la revendication 14, **caractérisée en ce que** ladite composition correspond à l'une des compositions définies dans l'une quelconque des revendications 15 à 33.

40. Procédé suivant la revendication précédente, **caractérisée en ce que** la quantité de composition appliquée topiquement sur la surface des épidermes en culture va de 0.5 µl à 10 µl par cm² de surface d'épiderme reconstruit.

41. Procédé suivant l'une quelconque des revendications 1 à 14 et 39 à 40, **caractérisé en ce que** l'épiderme reconstruit est le modèle EPISKIN^{TM}.

42. Equivalent de peau ou peau reconstruite contenant au moins un dérivé de la famille du céramide 5.5 de formule (I) telle que définie dans les revendications 1 à 4, dans laquelle X représente un atome d'hydrogène.
